# EUROPEAN PATENT APPLICATION

(11) **EP 4 597 512 A1**
(43) Date of publication of application: **06.08.2025**
(21) Application number: 23871067.7
(22) Date of filing: 28.09.2023
(51) Int. Cl.: G16H 40/63

(54) **DISPLAY METHOD FOR MEDICAL DEVICE, AND MEDICAL SYSTEM AND MEDICAL DEVICE**

(30) Priority: 30.09.2022 WO PCT/CN2022/123506
(71) Applicant: Air Force Medical University of PLA, Xi'an, Shaanxi 710032 (CN); Shenzhen Mindray Bio-Medical Electronics Co., Ltd., Shenzhen, Guangdong 518057 (CN)
(72) Inventor: DONG, Hailong, Xi'an, Shaanxi 710032 (CN); ZHANG, Haopeng, Xi'an, Shaanxi 710032 (CN); ZHAO, Guangchao, Xi'an, Shaanxi 710032 (CN); WAN, Congying, Shenzhen, Guangdong 518057 (CN); ZHOU, Xiaoyong, Shenzhen, Guangdong 518057 (CN); HUANGFU, Yong, Shenzhen, Guangdong 518057 (CN); HUANG, Chenghua, Shenzhen, Guangdong 518057 (CN)
(74) Representative: KIPA AB
(86) International application number: PCT/CN2023/122805
(87) International publication number: WO 2024/067843

(57) **Abstract**

A display method for a medical device, and a medical system and a medical device. The display method for a medical device comprises: acquiring medical data of a medical object, which medical data is provided by a monitoring device; acquiring data of control parameters of a treatment device for providing treatment for the medical object, wherein the treatment device is used for providing treatment for the medical object on the basis of the control parameters, so as to affect the medical data of the medical object; determining groups of physiological parameters and the control parameters, wherein each of the groups comprises at least one physiological parameter, and at least one control parameter that can affect the physiological parameter; providing a user interaction interface, and displaying, on the user interaction interface and in an associated manner, medical data in the same group and an operation interface for adjusting the control parameters; and when an adjustment instruction for the control parameters is acquired, feeding the adjustment instruction back to the treatment device. Medical data and control parameters are displayed in groups, such that the working efficiency of medical staff can be improved.

## Description

### TECHNICAL FIELD

The disclosure generally relates to a technical field of medical device, and more specifically to a display method for a medical device, a medical system, and a medical device.

### BACKGROUND

When performing anesthesia, a doctor needs to pay attention to various physiological parameters of patient, so as to ensure a safety of surgery and anesthesia, as well as needs for adjusting life support and drug injection according to a patient condition. In traditional anesthesia, physiological parameters that doctor needs to pay attention to, and life support and drug that need to be adjusted, are often on different devices, which causes a workflow of doctor to switch between interaction interfaces of multiple devices, thus resulting a lot of inconvenience to the doctor. For example, when a doctor observes on a monitor that physiological parameters of patient are not within an expected range and need to be adjusted, the doctor needs to turn to an anesthesia machine for adjusting inhaled anesthetic drugs and ventilation, or turn to a syringe pump for adjusting intravenous drugs. The workflow of doctor switches between multiple devices, which not only affects efficiency but also increases a potential risk of misjudgment and misoperation.

One possible solution is to bring display interfaces of each device closer together through screen mapping, or to display information from multiple devices simultaneously on a large screen, such that the doctor can switch between the interfaces more conveniently. However, this method divides information according to a device or a source of information, such as dividing information in a monitor area, an anesthesia machine area, and an injection pump area. The information is not organically combined together. The doctor still needs to find the information concerned to in each partition interface, and then turn to other areas to operate.

### SUMMARY

The summary introduces a series of concepts in simplified form that are further described in detail in the detailed description section. The summary section of this disclosure does not mean to attempt to limit key features and essential technical features of a technical solution claimed for protection, nor does it mean to attempt to determine a protection scope of a technical solution claimed for protection.

A first aspect of an embodiment of this disclosure provides a display method for a medical device, including:
acquiring medical data of a medical object which data is provided by a monitoring device, wherein the medical data includes data which is related to one or more physiological parameters of the medical object;
acquiring data for a control parameter of a treatment device, which device provides treatment to the medical object, wherein the treatment device is configured to provide the treatment to the medical object based on the control parameter, so as to affect the medical data of the medical object;
providing a user interaction interface; and on which interface associatively displaying, according to a grouping of the physiological parameter(s) and the control parameter, the medical data which is related to the physiological parameter(s) and an operation interface for adjusting the control parameter, which parameters are in a same group; wherein each group includes at least one physiological parameter and at least one control parameter which is capable of affecting medical data which is related to said physiological parameter; and
when an adjustment instruction for the control parameter is obtained, feedbacking the adjustment instruction to the treatment device.

A second aspect of an embodiment of this disclosure provides a medical system, including:
a monitoring device, which is configured to acquire medical data of a medical object, wherein the medical data includes data which is related to one or more physiological parameters of the medical object;
a treatment device, which is configure to provide treatment to the medical object based on a control parameter, so as to affect the medical data, wherein the monitoring device is in communicative connection with the treatment device;
a user interaction apparatus, which is a user interaction apparatus of the monitoring device, the treatment device or a third-party device; wherein the third-party device is in communicative connection with the monitoring device and the treatment device;
the user interaction apparatus is configured to:
   provide a user interaction interface;
   according to a grouping of the physiological parameter(s) and the control parameter, associatively display, on the user interaction interface, the medical data which is related to the physiological parameter(s) and an operation interface for adjusting the control parameter, which parameters are in a same group, or associatively display, on the user interaction interface, the medical data which is related to the physiological parameter(s) and data for the control parameter, which parameters are in a same group; and
   obtain an adjustment instruction for the control parameter, and feedback the adjustment instruction to the treatment device;
   wherein each group includes at least one physiological parameter and at least one control parameter which is capable of affecting medical data which is related to said physiological parameter.

A third aspect of an embodiment of this disclosure provides a display method for a medical device, including:
acquiring medical data of a medical object, wherein the medical data includes data which is related to one or more physiological parameters of the medical object;
acquiring data for a control parameter to provide treatment to the medical object, wherein the medical device is configured to provide the treatment to the medical object based on the control parameter, so as to affect the medical data of the medical object;
determining a grouping of the physiological parameter(s) and the control parameter, wherein each group includes at least one physiological parameter and at least one control parameter which is capable of affecting medical data which is related to said physiological parameter;
providing a user interaction interface; associatively displaying, on the user interaction interface, the medical data which is related to the physiological parameter(s) and an operation interface for adjusting the control parameter, which parameters are in a same group, or associatively displaying, on the user interaction interface, the medical data which is related to the physiological parameter(s) and the data for the control parameter, which parameters are in a same group;
when an adjustment instruction for the control parameter is obtained, adjusting the control parameter according to the adjustment instruction.

A fourth aspect of an embodiment of this disclosure provides a medical device, including:
a monitoring component, which is configured to acquire medical data of a medical object, wherein the medical data includes data which is related to one or more physiological parameters of the medical object;
a treatment component, which is configured to provide treatment to the medical object based on a control parameter, so as to affect the medical data;
a user interaction apparatus, which is configured to provide a user interaction interface;
a control apparatus, which is configured to:
   control the user interaction apparatus to associatively display, on the user interaction interface, according to a grouping of the physiological parameter(s) and the control parameter, the medical data which is related to the physiological parameter(s) and an operation interface for adjusting the control parameter, which parameters are in a same group; or to associatively display, on the user interaction interface, according to a grouping of the physiological parameter(s) and the control parameter, the medical data which is related to the physiological parameter(s) and data for the control parameter, which parameters are in a same group; wherein each group includes at least one physiological parameter and at least one control parameter which is capable of affecting medical data which is related to said physiological parameter; and
   when an adjustment instruction for the control parameter is obtained, adjust the control parameter according to the adjustment instruction.

According to the display method for a medical device, the medical system, and the medical device in embodiments of this disclosure, the medical data which is related to the physiological parameter(s) and an operation interface for adjusting the control parameter, which parameters are in a same group, are associatively displayed, which enables a user to more intuitively determine a current physiological state of the medical object and make more timely decisions on whether for adjusting the control parameter and how for adjusting the control parameter. At the same time, after the control parameter is adjusted, the physiological state of the medical object can be observed in time to improve work efficiency of doctor and reduce a probability of medical error.

### BRIEF DESCRIPTION OF THE DRAWINGS

In order to provide a clearer explanation of the technical solution in the embodiments of this disclosure, a brief introduction is given to the accompanying drawings required in the description of the embodiments. It is evident that the accompanying drawings in the following description are only some embodiments of this disclosure. For ordinary technical personnel in the art, other accompanying drawings can be obtained based on these drawings without any creative effort.
FIG. 1 is a schematic flow chart for a display method for a medical device according to an embodiment of this disclosure.
FIG. 2 is a schematic diagram for associatively displaying an operation interface for adjusting a control parameter, and medical data which is related to a physiological parameter, which parameters are in a same group, according to an embodiment of this disclosure.
FIG. 3 is a schematic diagram for a user interaction interface according to an embodiment of this disclosure.
FIG. 4 is a schematic block diagram for a medical system according to an embodiment of this disclosure.
FIG. 5 is a schematic flow chart for a display method for a medical device according to another embodiment of this disclosure.
FIG. 6 is a schematic flow chart for a display method for a medical device according to another embodiment of this disclosure.
FIG. 7 is a schematic block diagram for a medical device according to an embodiment of this disclosure.

### DESCRIPTION OF THE PREFERRED EMBODIMENTS

In order to make the purpose, technical solution, and advantages of this disclosure more apparent, the following refers to the attached drawings to describe in detail the exemplary embodiments according to this disclosure. Obviously, the described embodiments are only some of the embodiments of this disclosure, not all of them. It should be understood that this disclosure is not limited by the exemplary embodiments described here. Based on the embodiments described in this disclosure, all other embodiments obtained by those skilled in the art without creative labor should fall within the protection scope of this disclosure.

In the following description, a large number of specific details are provided to provide a more thorough understanding of this disclosure. However, it is evident to those skilled in the art that this disclosure can be implemented without one or more of these details. In other examples, in order to avoid confusion with this disclosure, some well-known technical features in the art have not been described.

It should be understood that this disclosure can be implemented in different forms and should not be limited to the embodiments proposed here. On the contrary, providing these embodiments makes the disclosure thorough and complete, and fully conveys the scope of this disclosure to those skilled in the art.

The purpose of the technical term used here is only to describe specific embodiments and is not a limitation of this disclosure. When used here, the singular forms of "a", "an", and "said/the" are also intended to include the plural form, unless the context clearly indicates another way. It should also be understood that the terms "include" and/or "comprise", when used in this disclosure, determine the presence of the features, integers, steps, operations, elements, and/or components, but do not exclude the presence or addition of one or more other features, integers, steps, operations, elements, and/or components. When used here, the term "and/or" includes any and all combinations of related listed items. In addition, this disclosure can be implemented in many different forms and is not limited to the embodiments described in this embodiment. The purpose of providing the following specific embodiments is to facilitate a clearer and more thorough understanding of the contents disclosed in this disclosure, wherein words indicating directions such as up, down, left, and right refer only to the positions of the structures shown in the corresponding drawings.

In order to fully understand this disclosure, a detailed structure is proposed in the following description to elucidate the technical solution proposed by this disclosure. The optional embodiments of this disclosure are described in detail below, however, in addition to these detailed descriptions, this disclosure may also have other embodiments.

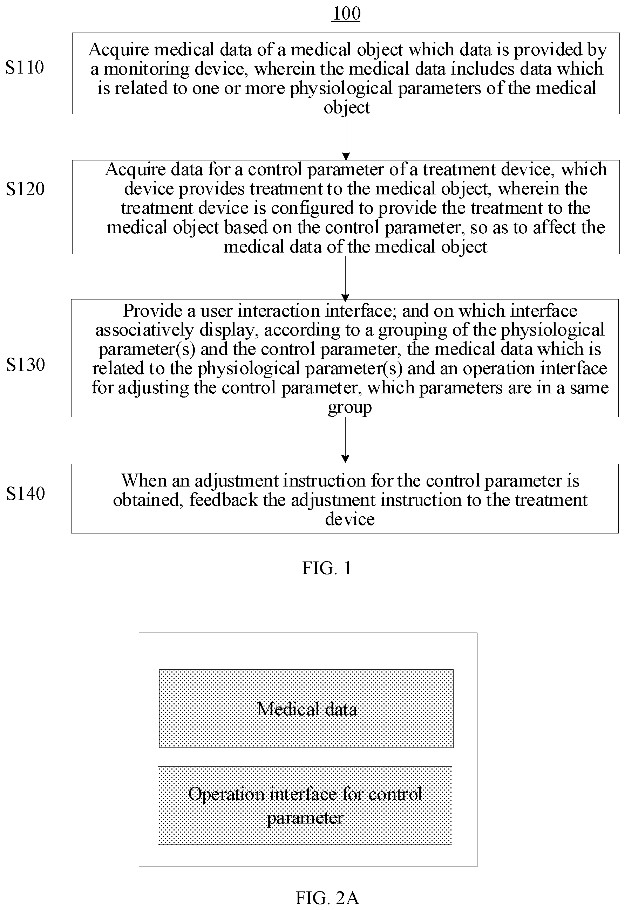

Hereinafter, a display method for a medical device according to an embodiment of this disclosure is described with reference to the accompanying drawings. First, refer to FIG. 1, which is a schematic flowchart for a display method 100 for a medical device according to an embodiment of this disclosure. As shown in FIG. 1, a display method 100 for a medical device according to an embodiment of this disclosure includes the following steps.

In step S110, acquire medical data of a medical object which data is provided by a monitoring device, wherein the medical data includes data which is related to one or more physiological parameters of the medical object. Specifically, the medical data may be monitoring data for one or more physiological parameters of the medical object, or monitoring result(s) which is(are) obtained based on monitoring data of one or more physiological parameters, or both.

In step S120, acquire data for a control parameter of a treatment device, which device provides treatment to the medical object, wherein the treatment device is configured to provide the treatment to the medical object based on the control parameter, so as to affect the medical data of the medical object.

In step S130, provide a user interaction interface; and on which interface associatively display, according to a grouping of the physiological parameter(s) and the control parameter, the medical data which is related to the physiological parameter(s) and an operation interface for adjusting the control parameter, which parameters are in a same group; wherein each group includes at least one physiological parameter and at least one control parameter which is capable of affecting medical data which is related to said physiological parameter.

In step S140, when an adjustment instruction for the control parameter is obtained, feedback the adjustment instruction to the treatment device.

The display method 100 for a medical device according to an embodiment of this disclosure can be implemented in a monitoring device, a treatment device, or a third-party device that is in communicative connection with the monitoring device and the treatment device. By providing a user interaction interface at a user interaction apparatus of the above-mentioned device, the medical data and the operation interface for adjusting the control parameter can be associatively displayed on the user interaction interface, such that the user can quickly find the operation interface associated with the medical data of interest and operate the operation interface, thereby completing an entire workflow of intraoperative monitoring, drug administration, life support, etc., more simply and smoothly.

The medical data of the medical object may include monitoring data for a physiological parameter of the medical object, and may also include a monitoring result which is obtained based on monitoring data for a physiological parameter. The monitoring data for a physiological parameter of the medical object includes but is not limited to value(s) of physiological parameter(s), such as an electrocardiogram parameter, a blood pressure parameter, a pulse oximetry parameter, a respiration parameter, a body temperature parameter, a cardiac output parameter, a carbon dioxide parameter, a respiratory mechanics parameter, a hemodynamic parameter, an oxygen metabolism parameter, an EEG parameter, a bi-spectral index parameter, and a microcirculation parameter. The monitoring result, which is obtained based on monitoring data for a physiological parameter, includes parameter alarm information which is obtained based on monitoring data for a physiological parameter, such as alarm information for exceeding a threshold, which information is triggered, when a value of physiological parameter exceeds a preset threshold. The monitoring result, which is obtained based on the monitoring data for a physiological parameter, can also be a physiological state of patient, which is obtained by integrating monitoring data of multiple physiological parameters. The physiological state of a patient can be at least one of an overall state, a state of a physiological system, a state of an organ, a state of a physiological part, and a state of a tissue, of the patient. The monitoring data for a physiological parameter can be obtained by a monitoring device. Exemplarily, the monitoring device may be any medical device that is capable of providing a monitoring function a for physiological parameter, including but not limited to, a monitor, a ventilator, an anesthesia machine, and the likes. The monitoring device is configured to monitor a physiological parameter of a medical object. For example, the physiological parameter, which is monitored by a monitor, includes but is not limited to a basic vital sign parameter, such as an electrocardiogram parameter, a heart rate parameter, a blood pressure parameter, and a blood oxygen parameter; the physiological parameter, which is detected by an anesthesia machine, includes but is not limited to a sedative muscle relaxation parameter, such as an EEG signal, a BIS (bi-spectral index) signal, and a neuromuscular electrical signal; the physiological parameter, which is detected by a ventilator, includes but is not limited to a respiratory mechanics parameter, such as an airway pressure, a respiratory flow rate, and a respiratory rate.

When the display method 100 for a medical device is implemented in a monitoring device itself, the monitoring device is capable of acquiring monitoring data for a physiological parameter, through a sensor which is equipped by itself, processing and displaying said data. When the display method 100 for a medical device is implemented in a treatment device or a third-party device, the treatment device or the third-party device is capable of acquiring monitoring data for a physiological parameter, which data is acquired by a monitoring device in real time, through a communication connection with the monitoring device.

The treatment device is configured to provide treatment to the medical object. Treatment refers to a process of intervening or changing a specific physiological parameter. A treatment method that the treatment device is capable of providing, includes but is not limited to medication administration, respiratory support, etc., that is, the treatment device mainly includes a ventilation device and a drug delivery device. The ventilation device includes a ventilator, an anesthesia machine, etc., while the drug delivery device includes an anesthesia machine, an infusion pump, etc. Wherein, the ventilation device is responsible for providing respiration and oxygenation support to the medical object; the drug delivery device is responsible for delivering drugs to the medical object, which drugs include but are not limited to volatile anesthetics delivered by inhalation, sedatives delivered intravenously, analgesics, muscle relaxants, pressor drugs, vasodilators, colloids, liquids, etc.

It is understandable that some medical devices can both monitor the physiological parameter and provide treatment to the medical object, thus combining the functions of a monitoring device and a treatment device. For example, while providing respiratory support to the medical object, the ventilator can also acquire a respiratory mechanics parameter of the medical object; while releasing anesthetic gas and providing respiratory support to the medical object, the anesthesia machine can also acquire a sedation and muscle relaxation parameter of the medical object. Therefore, the ventilator and the anesthesia machine can be realized as both monitoring device and treatment device.

The treatment device needs to provide treatment to the medical object based on a certain control parameter. The control parameter is usually allowed to be adjusted by medical personnel, and the medical personnel can affect the medical data of the medical object by adjusting a specified control parameter. Wherein, the control parameter of the ventilator includes a ventilation parameter, such as a pressure and a flow rate of a respiratory gas; a control parameter of an anesthesia machine, in addition to a ventilation parameter, further includes a control parameter for drug administration, such as a flow rate and a concentration of an anesthetic gas; a control parameter of an infusion pump mainly includes a control parameter for drug administration, such as an infusion rate, etc. Medical staff usually determine whether for adjusting the control parameter of the treatment device based on the monitoring data for a physiological parameter or a monitoring result of the medical object, and observe a change in a relevant physiological parameter of the medical object in real time, when adjusting the control parameter of the treatment device, so as to avoid operational errors.

Therefore, embodiments of this disclosure integrate and associatively display related physiological parameter and control parameter, which enables medical staff to more intuitively determine a current physiological state of the medical object, make more timely decisions on whether for adjusting the control parameter and how for adjusting the control parameter, more conveniently find an operation interface for the control parameter, and observe a reaction of the medical object in more real time, thereby improving work efficiency of medical staff and reducing a probability of medical error. In order to integrate a physiological parameter and a control parameter according to a user requirement, it is necessary to determine a grouping of the physiological parameter and the control parameter, each group includes at least one physiological parameter, and at least one control parameter that is capable of affecting medical data which is related to said at least one physiological parameter. Determining the grouping of the physiological parameter and the control parameter which is related to medical data can establish association(s) between different types of physiological parameter and of control parameter.

Specifically, the physiological parameter and the control parameter can be grouped according to an actual clinical requirement, a physiological parameter that medical staff need to pay attention to and a control parameter that need to be adjusted, in a certain scenario, can be classifies into one group to optimize a workflow of medical staff.

For example, the physiological parameter and the control parameter may be grouped according to a physiological state, wherein at least one physiological parameter which reflects a target physiological state and at least one control parameter which is capable of affecting the target physiological state may be classified into one group. Taking an application in anesthesia for example, during the anesthesia process, medical staff need to focus on an anesthesia state, a hemodynamic state and an oxygenation state of the medical object, and adjust a control parameter which is related to each state when necessary. Therefore, the target physiological state may include at least one of the anesthesia state, the oxygenation state and the hemodynamic state.

Wherein, the physiological parameter which reflects the anesthesia state specifically includes physiological parameter(s) which reflects(reflect) a sedation state, an analgesia state and a muscle relaxation (muscle relaxation) state, of the medical object. Exemplarily, the physiological parameter which reflects the sedation state (referred to as a sedation parameter), includes but is not limited to, a BIS (Bi-spectral Index), an entropy index, an MAC (Minimum Alveolar Concentration) monitoring value, an EEG signal, an EEG spectrum, or a power spectrum, etc.; the physiological parameter which reflects the analgesia state (referred to as an analgesia parameter) includes but is not limited to an ANI index, an ECG variability, etc.; the physiological parameter which reflects the muscle relaxation state (referred to as a muscle relaxation parameter) includes but is not limited to TOF, PTC, etc. The control parameter which is capable of affecting the anesthesia state includes a control parameter for drug administration of the anesthesia machine and of the infusion pump. The control parameter for drug administration of the anesthesia machine include a delivery setting parameter of inhaled anesthetic drug, and the inhaled anesthetic drug includes N2O, Hal (halothane), Iso (isoflurane), Sev (sevoflurane), Enf (enflurane), Des (desflurane), etc.; the control parameter for drug administration of the infusion pump includes a delivery setting parameter of intravenous sedative (such as propofol, etc.), intravenous analgesic (such as opioids, etc.), intravenous muscle relaxant (such as rocuronium, etc.), etc.

Exemplarily, the physiological parameter which reflects the hemodynamic state includes, but is not limited to, a hemodynamic parameter of a cardiovascular system, such as electrocardiogram, heart rate, blood pressure, cardiac output, preload and afterload, etc. The control parameter which is capable of affecting the hemodynamic state includes a control parameter for drug administration of the anesthesia machine and of the infusion pump, such as a delivery setting parameter of a vasoactive drug, a pressor drug, and etc.

Exemplarily, the physiological parameter which reflects the oxygenation state includes blood oxygen, CO₂ content, a ventilation-perfusion ratio, an oxygenation index, tissue oxygen saturation, etc. The control parameter which is capable of affecting the oxygenation state includes a ventilation control parameter of the anesthesia machine or of the ventilator, such as a ventilation setting parameter, for example a gas flow rate, oxygen concentration, etc.

In another embodiment, the physiological parameter and the control parameter are grouped in such a way that at least one physiological parameter which reflects a target physiological structure and at least one control parameter which is capable of affecting the target physiological structure are classified into one group. Wherein, the target physiological structure includes a target physiological system, such as a respiratory system, a metabolic system, a circulatory system, etc. The target physiological structure may also include a target physiological organ, such as a heart, a brain, and lungs. When diagnosing or treating a target physiological structure, medical personnel can view medical data which reflects a state of the target physiological structure, or adjust a control parameter that affects the target physiological structure.

In some embodiments, at least one physiological parameter which is related to a target surgery type, and at least one control parameter which is related to the target surgery type, can also be classified into one group, so that the medical data which is related to the target surgery type, and the operation interface for the control parameter which is related to the target surgery type are associatively displayed, so that medical staff can view the medical data that needs attention when performing the target surgery type, and adjust the control parameter which is related to the target surgery type.

In some embodiments, at least one physiological parameter which is related to a target medical condition, and at least one control parameter which is capable of affecting the at least one physiological parameter which is related to the target medical condition can also be classified into one group, so that the medical data which is related to the target medical condition and the operation interface for the control parameter which is related to the target medical condition are associatively displayed, making it convenient for medical staff to view the relevant medical data when diagnosing or treating the target medical condition, and for adjusting the relevant control parameter when intervention in these medical data is required.

In addition to automatically grouping the physiological parameter and the control parameter according to the above rules, the user can also set a grouping of the physiological parameter and the control parameter according to a requirement. Specifically, a group setting interface is provided, a group setting operation is acquired through the group setting interface, and at least one physiological parameter and at least one control parameter, which are selected by the group setting operation, are classified into one same group. A user can customize a combination of various physiological parameters and control parameters that require attention based on requirements of a patient, a medical condition, and a surgery, etc., and associatively display the medical data which reflects the medical condition of the patient with the corresponding control parameter, so as to obtain a user interaction interface that is more in line with the workflow of a specific type of patient. It should be noted that when grouping the physiological parameter and the control parameter, any one standard may be used for grouping, or two or more standards may be used for grouping, and a same physiological parameter or a same control parameter may exist in multiple groups.

Based on the grouping of the physiological parameter and the control parameter, a user interaction interface can be provided, and on the user interaction interface, medical data which is related to physiological parameter(s) in a same group, and an operation interface for adjusting a control parameter in said group are associatively displayed. When an adjustment instruction for the control parameter is obtained, the adjustment instruction is fed back to the treatment device, and the treatment device is controlled accordingly. The adjustment instruction for the control parameter may be directly obtained based on the operation interface displayed on the display interface. For example, the operation interface may be operated in a touchable manner to obtain the adjustment instruction for the control parameter. The adjustment instruction for the control parameter may also be obtained in other ways, for example, by voice or gesture.

On the user interaction interface, the medical data and the control parameter are presented in combination according to a grouping result. By associatively displaying the medical data which is related to the physiological parameter(s) and the operation interface for adjusting the control parameter, which parameters are in a same group, medical staff can observe the medical data and the corresponding control parameter at the same time, and intuitively determine a current physiological state of the medical object according to the medical data. When the medical data does not satisfy a preset requirement, for example, the monitoring data for a physiological parameter is not within an expected range, it is possible to make timely decisions on whether for adjusting the control parameter and how for adjusting the control parameter. At the same time, after the control parameter is adjusted, a reaction of the physiological state of the medical object can be observed in time, thereby improving work efficiency of doctor and reducing a probability of medical error.

Wherein, the user interaction interface is displayed on a user interaction apparatus, and the user interaction apparatus can be a user interaction apparatus of a monitoring device, a treatment device, or a third-party device that is in communicative connection with the monitoring device and the treatment device, and the user interaction apparatus includes a display. Each group of medical data which is related to a physiological parameter and operation interface for adjusting a control parameter is displayed on the same user interaction interface. Different groups of medical data which is related to the physiological parameter and operation interface for adjusting a control parameter can be displayed on a same user interaction interface or on different user interaction interfaces. For example, the medical data which is related to the hemodynamic state and an operation interface for adjusting the control parameter can be displayed on a user interaction apparatus of the monitor; the medical data which is related to the oxygenation state and an operation interface for adjusting the control parameter can be displayed on a user interaction apparatus of the anesthesia machine, or can also be displayed on the user interaction apparatus of the monitor.

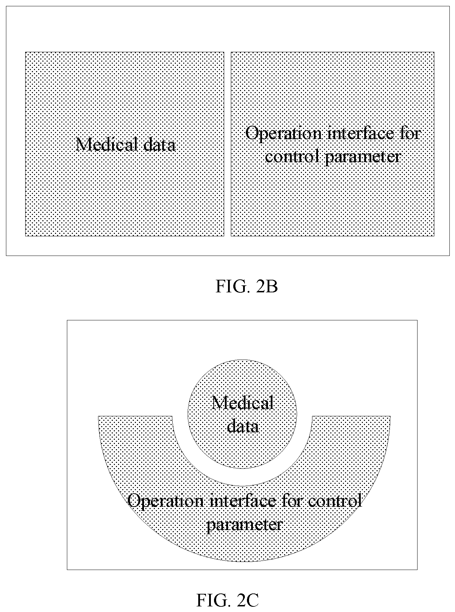

Exemplarily, the user interaction interface may include at least one display unit, each display unit corresponds to one group, and is configured to display medical data which is related to the physiological parameter in said group and an operation interface for the control parameter in said group. Exemplarily, in each display unit, the medical data and the operation interface for adjusting the control parameter in the same group may be displayed adjacent to each other, so that the user can intuitively observe a related set of medical data and control parameter. The adjacent display mode includes, but is not limited to, a longitudinal adjacent display as shown in FIG. 2A, a transverse adjacent display as shown in FIG. 2B , a circular adjacent display as shown in FIG. 2C, and the likes.

In another embodiment, a method for associatively displaying the medical data which is related to the physiological parameter(s) and an operation interface for adjusting the control parameter, which parameters are in a same group, includes: displaying the medical data which is related to the physiological parameter(s), and when a selection instruction for the medical data within a target group is received, displaying an operation interface for adjusting the control parameter, which control parameter is also within the target group in response to the received selection instruction. Exemplarily, at this time, the operation interface for the control parameter can be displayed in the form of a pop-up window in an adjacent area of the medical data.

In this embodiment, when the user does not need for adjusting the control parameter, the operation interface for the control parameter is hidden and only the medical data is displayed, thereby making a layout of the user interaction interface more concise. When it is necessary to affect the related medical data by adjusting the control parameter, the user can select the medical data of interest to call out the operation interface for the corresponding control parameter. The operation interface for the control parameter displayed at this time can be the operation interface of all control parameters in the target group, so that the user can select control parameter(s) that need(s) to be adjusted; or, the operation interface for the control parameter displayed at this time can also be the operation interface for control parameters which is(are) directly related to the selected medical data, that is, the operation interface for the control parameter(s) of the selected medical data can be directly intervened to prompt the user of the control parameter(s) that currently need to be adjusted.

Optionally, the medical data within a target group can be displayed by default on the user interaction interface, and when the medical data within the target group is determined to not satisfy a preset requirement, for example, when the monitoring data for a physiological parameter exceeds a preset range, or when a parameter alarm occurs, an operation interface for adjusting the control parameter within the target group is displayed. On the contrary, if the monitoring data of all physiological parameters in the target group are within the normal range, the user may not need for adjusting the control parameter, and thus the operation interface for the control parameter in the target group may not be displayed.

In some embodiments, whether the control parameter need to be adjusted can be determined according to the medical data, and when the control parameter is determined to need to be adjusted, indication information, which indicates that the control parameter need to be adjusted, is outputted. The way of outputting the indication information includes, but is not limited to, displaying the indication information that the control parameter needs to be adjusted on the user interaction interface.

For example, when the medical data related to a physiological parameter that is in a target group is determined to exceed a preset range, indication information may be displayed in the display unit which corresponds to the target group to indicate the user for adjusting the control parameter which is also in the target group. At this time, the medical data which exceeds the preset range may also be highlighted to indicate the user to pay attention to the abnormal medical data.

Furthermore, when a certain medical data related to a physiological parameter within a target group is determined to be abnormal, a target control parameter that can intervene in the medical data can be determined from a control parameter within the target group, and indication information, which indicates that the target control parameter needs to be adjusted, can be outputted to indicate the user for adjusting the target control parameter. This indication information can provide a reference for user, and can especially assist inexperienced medical personnel in determining, when abnormal medical data exists, which control parameter can be adjusted to improve the abnormal medical data.

Furthermore, an adjustment manner for the control parameter may be determined according to the medical data, and indication information about the adjustment manner may be outputted. Wherein, the adjustment manner for adjusting the control parameter includes but is not limited to increasing or decreasing a dosage of inhaled anesthetic drug or intravenous drug, increasing or decreasing a flow rate of respiratory gas, etc. This indication information can further assist the user in deciding how for adjusting the control parameter to improve the abnormal medical data.

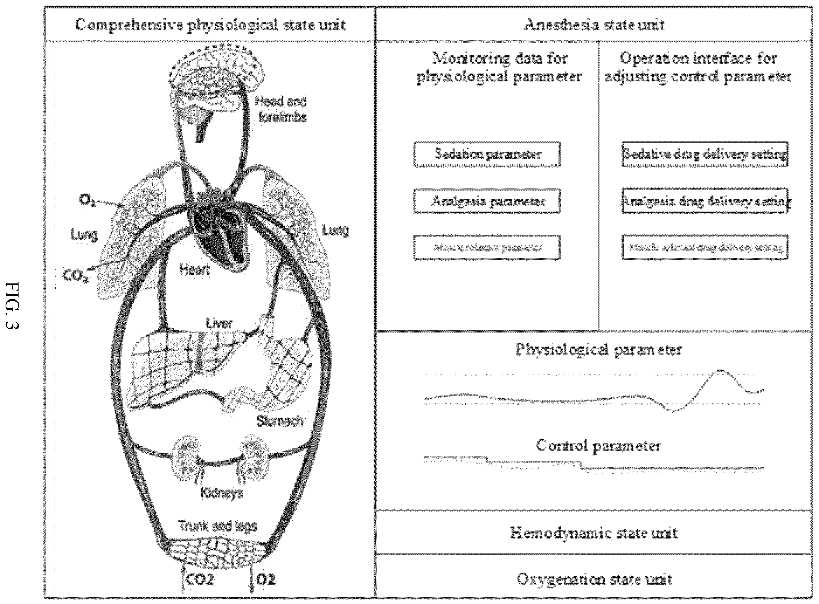

In some embodiments, a trend of change for the medical data may also be displayed together with the operation interface for adjusting the control parameter on the user interaction interface. Refer to FIG. 3, which shows an exemplary user interaction interface used in an anesthesia process. The user interaction interface currently displays a display unit which corresponds to an anesthesia state, and the user can switch said display unit to a display unit which corresponds to an oxygenation state or a display unit which corresponds to a hemodynamic state. In the display unit which corresponds to the anesthesia state, a sedation parameter, an analgesia parameter and a muscle relaxant parameter which reflect the anesthesia state, as well as an operation interface for a sedative drug delivery setting, an analgesia drug delivery setting and a muscle relaxant drug delivery setting which settings are capable of affecting the anesthesia state are specifically displayed. In addition, in the display unit which corresponds to the anesthesia state, a trend of change for a physiological parameter which reflects the anesthesia state is also displayed. The user can refer to the trend of change for the physiological parameter which reflects the anesthesia state, so as to understand changes in the anesthesia state of the user during the anesthesia process.

Exemplarily, when an adjustment instruction for a control parameter is received, an event mark which corresponds to the adjustment instruction can also be displayed on the trend of change for the medical data, so as to indicate that the user adjusted the related control parameter at a time point which corresponds to the event mark, thereby facilitating the user to understand a reason for the change in the medical data. Furthermore, in response to a selection instruction for an event mark, descriptive information for the adjustment instruction which is related to the event mark may be displayed, including but not limited to a type of control parameter that has been adjusted, an adjustment manner, etc.

Furthermore, a trend of change for the control parameter and a trend of change for the medical data may be displayed adjacent to each other, so as to reflect an adjustment process of the control parameter. For example, in the display unit which corresponds to the anesthesia state shown in FIG. 3, the trend of change for the control parameter which is related to the anesthesia state is also displayed. In one embodiment, the displayed trend of change for the control parameter may be the trend of change for an adjusted control parameter; correspondingly, the displayed trend of change for the medical data may be the trend of change for the medical data which is related to the adjusted control parameter. For example, if the delivery setting parameter of the intravenous sedative drug, such as propofol, is adjusted during anesthesia, the trend of change for the delivery setting parameter of the intravenous sedative drug can be displayed in the display unit which corresponds to the anesthesia state, and the trend of change for the sedation parameter, such as BIS, etc., which parameter reflects the sedation state, can be displayed accordingly. By comparing the trends of change of the medical data and of the control parameter, the user can determine a responsiveness of medical object to drug.

Continuing to refer to FIG. 3, in some embodiments, a physiological structure diagram may also be displayed on the user interaction interface. The physiological structure diagram includes a plurality of physiological structures, and the physiological structure may include a physiological organ or a physiological system. The physiological structure diagram can be used to reflect a comprehensive physiological state of medical object. For example, when an abnormality exists in the medical data which is related to a certain physiological structure, the corresponding physiological structure in the physiological structure diagram may be highlighted.

In addition, the physiological structure diagram can also be used to select the grouping of the physiological parameter and the control parameter which is displayed on the current user interaction interface. Specifically, a selection instruction for a target physiological structure in the physiological structure diagram may be received, and in response to the received selection instruction, monitoring data for a physiological parameter and an operation interface for adjusting a control parameter are displayed, which parameters are in a group which is related to the target physiological structure. For example, in the physiological structure diagram shown in FIG. 3, a currently selected target physiological structure is the head, and the display unit which is presented on the right side of the physiological structure diagram is a display unit which is related to an anesthesia state. Similarly, if a selection instruction for a respiratory system in the physiological structure diagram is received, a display unit which is related to an oxygenation state may be displayed; if a selection instruction for a circulatory system in the physiological structure diagram is received, a display unit which is related to a hemodynamic state may be displayed. Conversely, after determining a currently displayed group by other means, a physiological structure which is related to said group can be indicated in the physiological structure diagram. For example, after determining that a display unit currently displayed is related to the anesthesia state, the head position in the physiological structure diagram is highlighted.

In summary, the display method 100 for a medical device in the embodiment of this disclosure associatively displays the medical data which is related to the physiological parameter(s) and the operation interface for adjusting the control parameter, which parameters are in the same group, which enables the user to more intuitively determine a current physiological state of the medical object and make more timely decisions on whether for adjusting the control parameter and how for adjusting the control parameters. At the same time, after the control parameter is adjusted, the physiological state of the medical object can be observed in time to improve work efficiency of doctor and reduce a probability of medical error.

On the other hand, an embodiment of this disclosure provides a medical system, which can be used to implement the above-mentioned display method 100 for a medical device.

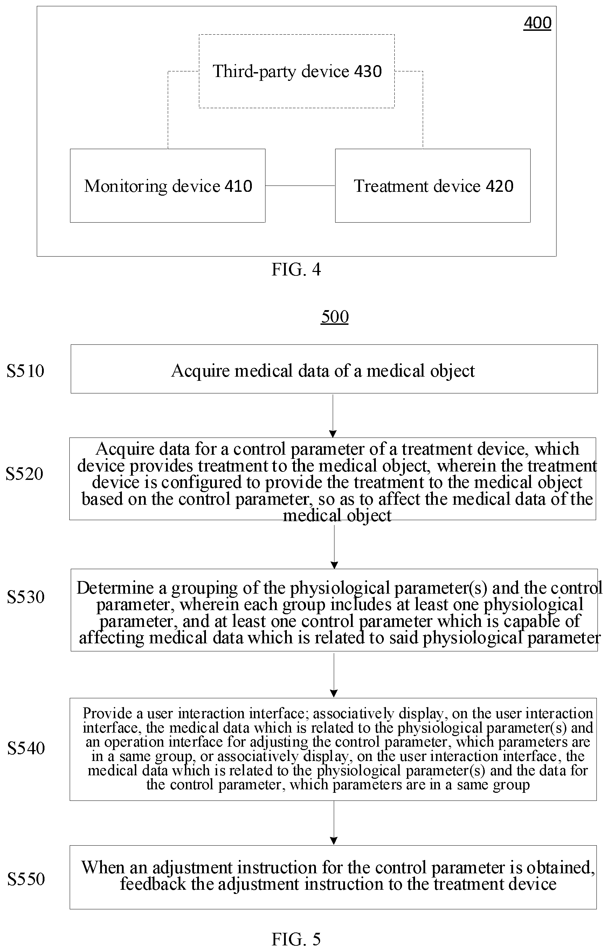

Referring to FIG. 4, a medical system 400 includes: a monitoring device 410, which is configured to acquire medical data of a medical object, wherein the medical data includes data which is related to one or more physiological parameters of the medical object, such as monitoring data for a physiological parameter of the medical object and/or a monitoring result which is obtained based on monitoring data; a treatment device 420, which is configured to provide treatment for the medical object based on a control parameter, so as to affect the physiological parameter; a user interaction apparatus, which is a user interaction apparatus of the monitoring device 410, the treatment device 420, or a third-party device 430. When the user interaction apparatus is a user interaction apparatus of the monitoring device 410 or the treatment device 420, the monitoring device 410 and the treatment device 420 are in communicative connection with each other. When the user interaction apparatus is a user interaction apparatus of a third-party device 430, the third-party device 430 is in communicative connection with the monitoring device 410 and the treatment device 420. The user interaction apparatus is configured to provide a user interaction interface; on the user interaction interface associatively display, according to a grouping of the medical data which is related to the physiological parameter and a control parameter, the medical data which is related to the physiological parameter, and an operation interface for adjusting the control parameter, which parameters are in the same group; obtain an adjustment instruction for the control parameter, and feedback obtained adjustment instruction to the treatment device 420; wherein each group includes at least one physiological parameter and at least one control parameter which is capable of affecting medical data which is related to said physiological parameter.

Exemplarily, the monitoring device 410 can be any medical device that can provide physiological parameter monitoring function. The monitoring device 410 mainly includes a processor, a user interaction apparatus and a sensor. The processor controls the sensor to acquire physiological parameter data and controls the user interaction apparatus to present the physiological parameter data. The sensor and the processor can be connected via a wired communication protocol or a wireless communication protocol to exchange data. The monitoring device 410 includes but is not limited to a monitor, a ventilator, an anesthesia machine, etc. Different monitoring devices 410 can monitor different physiological parameters of the medical object. For example, the monitor can monitor a basic vital sign parameter, the ventilator can monitor a respiratory parameter, the anesthesia machine can monitor a physiological parameter which reflects the anesthesia state, such as a sedation parameter, an analgesia parameter, and a muscle relaxation parameter.

The treatment device 420 is configured to provide treatment to the medical object to intervene in or change specific physiological parameter(s). The treatment method which is capable of being provided by the treatment device 420, includes but is not limited to, drug administration, respiratory support, etc., that is, the treatment device 420 mainly includes two types of device, that is, a ventilation device and a drug delivery device. Wherein, the ventilation device is responsible for providing respiration and oxygenation support to the medical object; the drug delivery device is responsible for delivering drugs to the medical object. The ventilation device mainly includes a processor, a user interaction apparatus and a ventilation unit. The processor controls the ventilation unit to provide mechanical ventilation for the medical object. The drug delivery device includes a processor, a user interaction apparatus and a drive mechanism. The processor controls the drive mechanism to inject the drug into a body of the medical object. Exemplarily, the ventilation device includes a ventilator or an anesthesia machine, and the drug delivery device includes an anesthesia machine or an infusion pump, that is, the anesthesia machine can be both a ventilation device and a drug delivery device.

In one example, the medical system 400 includes a monitor, an anesthesia machine, and an infusion pump, and has functions of physiological parameter monitoring, respiratory support, and drug delivery, and can achieve collaborative work of multiple devices. The user interaction apparatus of the medical system 400 may be a user interaction apparatus of a monitor or a user interaction apparatus of an anesthesia machine, which is configured to group and fuse the monitoring data for a physiological parameter which is acquired by the monitor, the data of physiological parameter which is acquired by the anesthesia machine, the ventilation control interface of the anesthesia machine, the drug delivery setting interface of the anesthesia machine, and the drug delivery setting interface of the infusion pump, for displaying. When the user interaction apparatus is a user interaction apparatus of a monitor, the monitor is connected with the anesthesia machine and the infusion pump, so as to obtain data for a physiological parameter which is acquired by the anesthesia machine, and transmitting an adjustment instruction for a control parameter to the anesthesia machine and the infusion pump. When the user interaction apparatus is a user interaction apparatus of an anesthesia machine, the anesthesia machine is in communicative connection with the monitor and the infusion pump, so as to obtain data for a physiological parameter which is acquired by the monitor, and to transmit an adjustment instruction for a control parameter to the infusion pump.

Optionally, the device which provides the user interaction apparatus may also be a third-party device 430 that is different from the monitoring device 410 and the treatment device 420. The third-party device 430 may be another electronic device, which is in communicative connection with the monitoring device 410 and the treatment device 420 and has data receiving, data processing and data displaying functions, including but not limited to, a central station or a mobile terminal, such as a mobile phone, a tablet computer, and a personal computer. The third-party device 430 obtains the medical data which is provided by the monitoring device 410 through a communication connection with the monitoring device 410, obtains the data for the control parameter of the treatment device 420 through a communication connection with the treatment device 420, groups and fuses the two data for displaying, and feedbacks a received adjustment instruction for the control parameter to the treatment device 420.

Exemplarily, a communication method between the monitoring device 410 and the treatment device 420, or a communication method between the third-party device 43 and the monitoring device 410 and the treatment device 420, includes but is not limited to Wi-Fi, Bluetooth, NFC, ZigBee, ultra-wideband UWB or 2G, 3G, 4G, 5G and another mobile communication method. The communication method may also be a wired communication method via a cable.

The monitoring device 410, the treatment device 420 or the third-party device 430 is further configured to group the physiological parameter and the control parameter. The device used for grouping can be a device that provides a user interaction interface among the monitoring device 410, the treatment device 420 and the third-party device 430. For example, the monitoring device 410 obtains medical data for a treated object which data is related to one or more physiological parameters, obtains data for a control parameter from the treatment device 420, groups the physiological parameter and the control parameter, and provides a user interaction interface on its own user interaction apparatus, so as to associatively display medical data which is related to the physiological parameter and an operation interface for adjusting the control parameter, which parameters are within the same group. A device used for grouping may also be different from a device for providing the user interaction interface, and the device used for grouping may transmit a grouping result to the device for providing the user interaction interface.

Wherein, each grouping may include: at least one physiological parameter which reflects a target physiological state, and at least one control parameter which is capable of affecting the target physiological state; at least one physiological parameter which reflects a target physiological system, and at least one control parameter which is capable of affecting the target physiological system; at least one physiological parameter which is related to a target surgical type, and at least one control parameter which is related to the target surgical type; or at least one physiological parameter which is related to a target medical condition, and at least one control parameter which is capable of affecting said at least one physiological parameter which is related to the target medical condition.

When the user interaction apparatus associatively displays the medical data which is related to the physiological parameter(s) and the operation interface for adjusting the control parameter, which parameters are in a same group, the medical data which is related to the physiological parameter(s) and the operation interface for adjusting the control parameter, which parameters are in the same group may be displayed adjacent to each other. Alternatively, the medical data may be displayed, and in response to a selection instruction for medical data related to the physiological parameter(s) within a target group, an operation interface for adjusting the control parameter, which control parameter is also within the target group may be displayed. The user interaction apparatus can also display the medical data, and when the medical data related to the physiological parameter(s) in a target group is determined to exceed a preset range, display an operation interface for adjusting the control parameter in the target group. For more specific details about the display of the grouped medical data and control parameter, please refer to the relevant description of the display method 100 for a medical device, which is not elaborated here.

The medical system 400 of the embodiment of this disclosure associatively displays the medical data which is related to the physiological parameter(s) and an operation interface for adjusting the control parameter, which parameters are in a same group, which enables efficient system operation of the monitoring device and the treatment device, enables a user to more intuitively determine a current physiological state of the medical object and make more timely decisions on whether for adjusting the control parameter and how for adjusting the control parameter. At the same time, after the control parameter is adjusted, the physiological state of the medical object can be observed in time to improve work efficiency of doctor and reduce a probability of medical error.

Referring FIG. 5, another aspect of this disclosure embodiment provides a display method 500 for a medical device, including following steps. In step S510, acquire medical data of a medical object, wherein the medical data is related to one or more physiological parameters of the medical object.

In step S520, acquire data for a control parameter of a treatment device, which device provides treatment to the medical object, wherein the treatment device is configured to provide the treatment to the medical object based on the control parameter, so as to affect the medical data of the medical object.

In step S530, determine a grouping of the physiological parameter(s) and the control parameter, wherein each group includes at least one physiological parameter and at least one control parameter which is capable of affecting medical data which is related to said physiological parameter.

In step S540, provide a user interaction interface; associatively display, on the user interaction interface, the medical data which is related to the physiological parameter(s) and an operation interface for adjusting the control parameter, which parameters are in a same group, or associatively display, on the user interaction interface, the medical data which is related to the physiological parameter(s) and the data for the control parameter, which parameters are in a same group.

In step S550, when an adjustment instruction for the control parameter is obtained, feedback the adjustment instruction to the treatment device.

The display method 500 for a medical device is substantially similar to the display method 100 for a medical device, except that, firstly, the medical data of the medical object is not limited to the monitoring data for a physiological parameter or the monitoring result which is obtained based on the monitoring data, but may also be other data which is capable of reflecting a characteristic or a state of the medical object. For example, the medical data may also be medical history data, nursing data, test data, etc. of the medical object. Secondly, on the user interaction interface, the medical data and the operation interface for adjusting the control parameter in the same group can be associatively displayed, or the medical data and the data for the control parameter in the same group can be associatively displayed. When the medical data and an operation interface are associatively displayed, the operation interface can be controlled by a touchable manner to obtain an adjustment instruction for adjusting the control parameter through the operation interface; when the medical data and the data for the control parameter are associatively displayed, the control parameter can be adjusted by voice, gesture or physical button, and the control parameter displayed on the display interface is updated in real time to facilitate a user for adjusting the control parameter by referring to the data for the control parameter displayed on the display interface. For more specific details of the display method 500 for a medical device, reference may be made to the relevant description of the display method 100 for a medical device, which is not elaborated here.

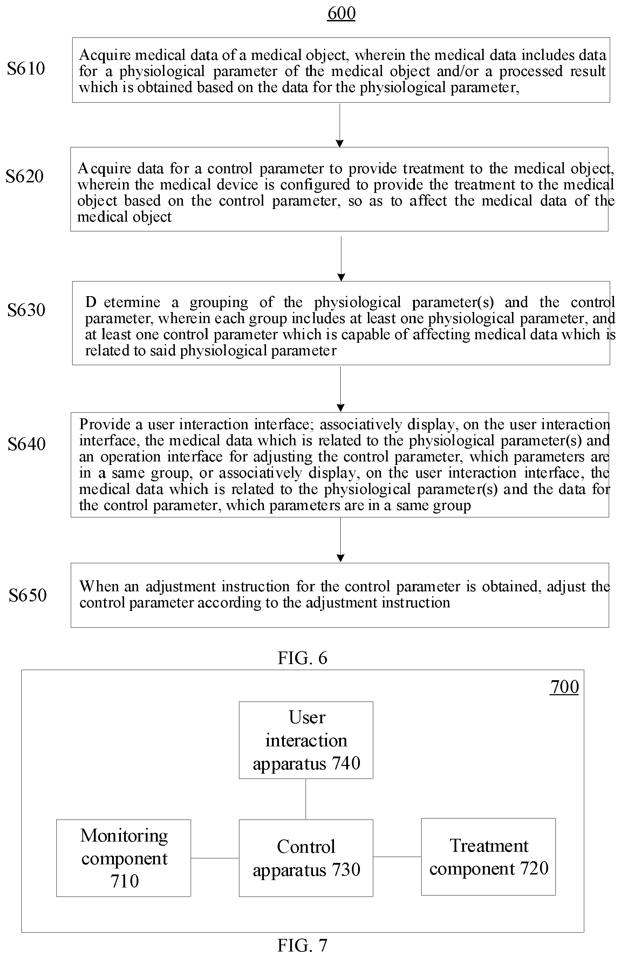

Referring to FIG. 6, another aspect of an embodiment of this disclosure provides a display method 600 for a medical device. As shown in FIG. 6, a display method 600 for a medical device includes following steps.

In step S610, acquire medical data of a medical object, wherein the medical data includes data for a physiological parameter of the medical object and/or a processed result which is obtained based on the data for the physiological parameter, that is, the medical data is data which is related to one or more physiological parameters.

In step S620, acquire data for a control parameter to provide treatment to the medical object, wherein the medical device is configured to provide the treatment to the medical object based on the control parameter, so as to affect the medical data of the medical object.

In step S630, determine a grouping of the physiological parameter(s) and the control parameter, wherein each group includes at least one physiological parameter and at least one control parameter which is capable of affecting medical data which is related to said physiological parameter.

In step S640, provide a user interaction interface; associatively display, on the user interaction interface, the medical data which is related to the physiological parameter(s) and an operation interface for adjusting the control parameter, which parameters are in a same group, or associatively display, on the user interaction interface, the medical data which is related to the physiological parameter(s) and the data for the control parameter, which parameters are in a same group.

In step S650, when an adjustment instruction for the control parameter is obtained, adjust the control parameter according to the adjustment instruction.

The display method 600 for a medical device is substantially similar to the display method 100 for a medical device, except that the display method 600 for a medical device involves a separate medical device that is used for both acquiring medical data of a medical object and providing treatment to the medical object based on the control parameter. Exemplarily, the medical device implementing the display method 600 for a medical device may be a ventilator or an anesthesia machine. The display method 600 of the medical device groups the medical data and control parameter, and associatively displays the medical data and the operation interface for adjusting the control parameter in the same group, which optimizes the user interaction interface of the medical device, improves work efficiency of doctor, and reduces a probability of medical error. For more specific details of the display method 600 for a medical device, reference may be made to the relevant description of the display method 100 for a medical device, which is not elaborated here.

Another aspect of an embodiment of this disclosure provides a medical device for implementing the above-mentioned display method 600 for a medical device. Referring FIG. 7, the medical device 700 includes: a monitoring component 710, which is configured to acquire medical data of a medical object, wherein the medical data includes data for a physiological parameter of the medical object, and/or a processed result which is obtained based on the data for the physiological parameter; a treatment component 720, which is configured to provide treatment for the medical object based on a control parameter, so as to affect the medical data; a user interaction apparatus 740, which is configured to provide a user interaction interface; a control apparatus 730, which is configured to: control the user interaction apparatus 740 to associatively display, on the user interaction interface, according to a grouping of the physiological parameter and the control parameter, the medical data and an operation interface for adjusting the control parameter, which are in a same group; or the medical data and data for the control parameter data, which are in a same group; and when an adjustment instruction for the control parameter is obtained, adjust the control parameter according to the adjustment instruction. Each group includes at least one physiological parameter, and at least one control parameter which is capable of affecting the medical data which is related to the physiological parameter.

In one embodiment, the medical device 700 includes a ventilator or an anesthesia machine, wherein the monitoring component 710 of the ventilator is configured to acquire data for a respiratory parameter of a medical object, and the treatment component 720 is configured to provide respiratory support for the medical object. The monitoring component 710 of the anesthesia machine is configured to acquire data for a physiological parameter of the medical object, which parameter reflects a responded anesthesia state, the treatment component 720 is configured to provide respiratory support and anesthetic drug delivery to the medical object. The medical device 700 may also be other medical devices with monitoring and treatment functions. Since the medical device 700 can implement the above-mentioned display method 600 for a medical device, it also has similar advantages.

Although exemplary embodiments have been described here with reference to the accompanying drawings, it should be understood that the aforementioned exemplary embodiments are only exemplary and are not intended to limit the scope of this disclosure to this extent. Ordinary technical personnel in this field can make various changes and modifications without deviating from the scope and spirit of this disclosure. All these changes and modifications are intended to be included within the scope of this disclosure as claimed in the attached claims.

Ordinary technical personnel in this field can realize that the units and algorithm steps of each example described in combination with the disclosed embodiments in this disclosure can be implemented in electronic hardware, or a combination of computer software and electronic hardware. Whether these functions are executed in hardware or software depends on the specific application and design constraints of the technical solution. Professional technicians may use different methods to achieve the described functions for each specific application, but such implementation should not be considered beyond the scope of this disclosure.

In the several embodiments provided in this disclosure, it should be understood that the disclosed devices and methods can be implemented in other ways. For example, the device embodiments described above are only illustrative. For example, the division of units is only a logical functional division, and there may be other division methods in actual implementation. For example, a plurality of units or components can be combined or integrated into another device, or some features can be ignored or not executed.

The description provided here provides a large number of specific details. However, it can be understood that embodiments of this disclosure can be practiced without these specific details. In some examples, well-known methods, structures, and techniques are not shown in detail to avoid blurring the understanding of this specification.

Similarly, it should be understood that in order to streamline this disclosure and assist in understanding one or more of the various aspects of this disclosure, in the description of exemplary embodiments of this disclosure, the various features of this disclosure are sometimes grouped together into a single embodiment, diagram, or description thereof. However, the method of this disclosure should not be interpreted as reflecting the intention that the claimed protection of this disclosure requires more features than those explicitly recorded in each claim. More precisely, as reflected in the corresponding claims, the inventive point is that the corresponding technical problem can be solved with less than all features of a single disclosed embodiment. Therefore, the claims following the specific implementation method are explicitly incorporated into the specific implementation method, where each claim itself serves as a separate embodiment of this disclosure.

Those skilled in the art will understand that, except where features are mutually exclusive, any combination can be configured to combine all features disclosed in this specification (including accompanying claims, abstracts, and drawings), as well as all processes or units of any method or device so disclosed. Unless otherwise explicitly stated, each feature disclosed in this specification (including accompanying claims, abstracts, and accompanying drawings) may be replaced by alternative features that provide the same, equivalent, or similar purpose.

In addition, those skilled in the art can understand that although some embodiments herein include certain features included rather than other features in other embodiments, the combination of features of different embodiments means that they fall into the scope of this disclosure and form different embodiments. For example, in the claims, any one of the claimed embodiments can be used in any combination.

The embodiments of various components of this disclosure can be implemented in hardware, software modules running on one or more processors, or a combination of them. Technicians in this field should understand that a microprocessor or digital signal processor (DSP) can be used in practice to implement some or all of the functions of some modules according to embodiments of this disclosure. This disclosure can also be implemented as a device program (such as a computer program and a computer program product) for executing some or all of the methods described herein. The program implementing this disclosure can be stored on a computer-readable medium or can take the form of one or more signals. Such signals can be downloaded from internet websites, provided on carrier signals, or in any other form.

It should be noted that the above embodiments illustrate this disclosure rather than limit it, and those skilled in the art can design alternative embodiments without departing from the scope of the attached claims. In the claims, any reference symbol between parentheses should not be constructed as a restriction on the claims. This disclosure can be implemented with the help of hardware including several different components and with the help of appropriately programmed computers. Among the unit claims that list several devices, several of these devices can be specifically embodied through the same hardware item. The use of words first, second, and third does not indicate any order. These words can be interpreted as names.

The above description is only for the specific implementation mode or explanation of the specific implementation mode of this disclosure. The protection scope of this disclosure is not limited to this. Any skilled person familiar with the technical field can easily think of changes or replacements within the technical scope disclosed in this disclosure, which should be included in the protection scope of the present application. The protection scope of this disclosure shall be subject to the protection scope of the claims.

## Claims

1. A display method for a medical device, **characterized in that**, comprising:
acquiring medical data of a medical object which data is provided by a monitoring device, wherein the medical data comprises data which is related to one or more physiological parameters of the medical object;
acquiring data for a control parameter of a treatment device, which device provides treatment to the medical object, wherein the treatment device is configured to provide the treatment to the medical object based on the control parameter, so as to affect the medical data of the medical object;
providing a user interaction interface; and on which interface associatively displaying, according to a grouping of the physiological parameter(s) and the control parameter, the medical data which is related to the physiological parameter(s) and an operation interface for adjusting the control parameter, which parameters are in a same group; wherein each group comprises at least one physiological parameter and at least one control parameter which is capable of affecting medical data which is related to said physiological parameter; and
when an adjustment instruction for the control parameter is obtained, feedbacking the adjustment instruction to the treatment device.

2. The method according to claim 1, **characterized in that**, the monitoring device comprises at least one of: a monitor, a ventilator and an anesthesia machine;
the treatment device comprises at least one of: a ventilator, an anesthesia machine and an infusion pump.

3. The method according to claim 2, **characterized in that**, the control parameter of the ventilator comprises a ventilation parameter;
the control parameter of the anesthesia machine comprises a ventilation parameter, and/or a control parameter for drug administration;
the control parameter of the infusion pump comprises a control parameter for drug administration.

4. The method according to claim 1, **characterized in that**, said each group comprises at least one physiological parameter which reflects a target physiological state, and at least one control parameter which is capable of affecting the target physiological state.

5. The method according to claim 1, **characterized in that**, said each group comprises at least one physiological parameter which reflects a target physiological structure, and at least one control parameter which is capable of affecting the target physiological structure; wherein the target physiological structure comprises a target physiological system and/or a target physiological organ.

6. The method according to claim 1, **characterized in that**, said each group comprises at least one physiological parameter which is related to a target surgery type, and at least one control parameter which is related to the target surgery type.

7. The method according to claim 1, **characterized in that**, said each group comprises at least one physiological parameter which is related to a target medical condition, and at least one control parameter which is capable of affecting said at least one physiological parameter which is related to the target medical condition.

8. The method according to claim 1, **characterized in that**, further comprising determining the grouping of the physiological parameter(s) and the control parameter;
wherein determining the grouping of the physiological parameter(s) and the control parameter, comprises:
providing a group setting interface, and obtaining a group setting operation through the group setting interface;
classifying into one same group at least one physiological parameter and at least one control parameter, which parameters are selected by the group setting operation.

9. The method according to claim 1, **characterized in that**, associatively displaying the medical data which is related to the physiological parameter(s) and an operation interface for adjusting the control parameter, which parameters are in a same group, comprises:
displaying, adjacent to each other, the medical data which is related to the physiological parameter(s) and the operation interface for adjusting the control parameter, which parameters are in the same group.

10. The method according to claim 1, **characterized in that**, associatively displaying the medical data which is related to the physiological parameter(s) and an operation interface for adjusting the control parameter, which parameters are in a same group, comprises:
displaying the medical data; and
in response to a selection instruction for the medical data related to the physiological parameter(s) that is(are) in a target group, displaying the operation interface for adjusting the control parameter, which control parameter is also in the target group.

11. The method according to claim 1, **characterized in that**, associatively displaying the medical data which is related to the physiological parameter(s) and an operation interface for adjusting the control parameter, which parameters are in a same group, comprises:
displaying the medical data; and
when the medical data related to the physiological parameter(s) that is(are) in a target group, is detected to exceed a preset range, displaying the operation interface for adjusting the control parameter, which control parameter is also in the target group.

12. The method according to claim 4, **characterized in that**, the target physiological state comprises an anesthesia state; and the control parameter which is capable of affecting the anesthesia state comprises a control parameter for drug administration of an anesthesia machine, and/or a control parameter for drug administration of an infusion pump.

13. The method according to claim 12, **characterized in that**, the physiological parameter which reflects the anesthesia state comprises physiological parameter(s) which reflects(reflect) a sedation state, an analgesia state and a muscle relaxation state of the medical object.

14. The method according to claim 4, **characterized in that**, the target physiological state comprises a hemodynamic state, and the control parameter which is capable of affecting the hemodynamic state comprises a control parameter for drug administration of an anesthesia machine, and/or a control parameter for drug administration of an infusion pump.

15. The method according to claim 4, **characterized in that**, the target physiological state comprises an oxygenation state, and the control parameter which is capable of affecting the oxygenation state comprises a ventilation control parameter of an anesthesia machine or a ventilation control parameter of a ventilator.

16. The method according to claim 1, **characterized in that**, further comprising:
associatively displaying a trend of change for the medical data which is related to the physiological parameter(s), together with said medical data and the operation interface for adjusting the control parameter, which parameters are in a same group.

17. The method according to claim 16, **characterized in that**, further comprising:
displaying, on the trend of change for the medical data, an event mark which corresponds to the adjustment instruction for the control parameter, when the adjustment instruction for the control parameter is received.

18. The method according to claim 17, **characterized in that**, further comprising:
in response to a selection instruction for the event mark, displaying descriptive information for adjustment instruction which information is related to the event mark.

19. The method according to claim 1, **characterized in that**, further comprising:
displaying a physiological structure diagram on the user interaction interface, wherein the physiological structure diagram comprises a plurality of physiological structures;
wherein associatively displaying the medical data which is related to the physiological parameter(s) and an operation interface for adjusting the control parameter, which parameters are in a same group, comprises:
receiving a selection instruction for a target physiological structure in the physiological structure diagram, and in response to the selection instruction, displaying the medical data which is related to the physiological parameter(s) and the operation interface for adjusting the control parameter, which parameters are in the same group, which group is related to the target physiological structure.

20. The method according to claim 1, **characterized in that**, further comprising:
determining whether the control parameter needs to be adjusted according to the medical data; and
outputting indication information which indicates that the control parameter needs to be adjusted, when it is determined that the control parameter needs to be adjusted.

21. The method according to claim 1, **characterized in that**, further comprising:
determining a target control parameter which needs to be adjusted according to the medical data; and
outputting indication information which indicates that the target control parameter needs to be adjusted.

22. The method according to claim 1, **characterized in that**, further comprising:
determining an adjustment manner for the control parameter according to the medical data; and
outputting indication information about the adjustment manner.

23. The method according to claim 1, **characterized in that**, the medical data comprises monitoring data for the physiological parameter(s) of the medical object, and/or a monitoring result which is obtained based on monitoring data.

24. A medical system, **characterized in that**, comprising:
a monitoring device, which is configured to acquire medical data of a medical object, wherein the medical data comprises data which is related to one or more physiological parameters of the medical object;
a treatment device, which is configure to provide treatment to the medical object based on a control parameter, so as to affect the medical data;
a user interaction apparatus, which is a user interaction apparatus of the monitoring device, the treatment device or a third-party device; wherein when the user interaction apparatus is the user interaction apparatus of the monitoring device or of the treatment device, the monitoring device and the treatment device are in communicative connection with each other; when the user interaction apparatus is the user interaction apparatus of the third-party device, the third-party device is in communicative connection with the monitoring device and the treatment device;
wherein the user interaction apparatus is configured to:
provide a user interaction interface;
according to a grouping of the physiological parameter(s) and the control parameter, associatively display, on the user interaction interface, the medical data which is related to the physiological parameter(s) and an operation interface for adjusting the control parameter, which parameters are in a same group, or associatively display, on the user interaction interface, the medical data which is related to the physiological parameter(s) and data for the control parameter, which parameters are in a same group; and
obtain an adjustment instruction for the control parameter, and feedback the adjustment instruction to the treatment device;
wherein each group comprises at least one physiological parameter and at least one control parameter which is capable of affecting medical data which is related to said physiological parameter.

25. The system according to claim 24, **characterized in that**, the monitoring device comprises at least one of: a monitor, a ventilator and an anesthesia machine;
the treatment device comprises at least one of: a ventilator, an anesthesia machine and an infusion pump.

26. The system according to claim 24, **characterized in that**, each said group comprises at least one physiological parameter which reflects a target physiological state, and at least one control parameter which is capable of affecting the target physiological state.

27. The system according to claim 24, **characterized in that**, each said group comprises at least one physiological parameter which reflects a target physiological system, and at least one control parameter which is capable of affecting the target physiological system.

28. The system according to claim 24, **characterized in that**, each said group comprises at least one physiological parameter which is related to a target surgery type, and at least one control parameter which is related to the target surgery type.

29. The system according to claim 24, **characterized in that**, each said group comprises at least one physiological parameter which is related to a target medical condition, and at least one control parameter which is capable of affecting said at least one physiological parameter which is related to the target medical condition.

30. The system according to claim 24, **characterized in that**, in order to associatively display, on the user interaction interface, the medical data which is related to the physiological parameter(s) and an operation interface for adjusting the control parameter, which parameters are in a same group, the user interaction apparatus is further configured to:
display, adjacent to each other, the medical data which is related to the physiological parameter(s) and the operation interface for adjusting the control parameter, which parameters are in the same group.

31. The system according to claim 24, **characterized in that**, in order to associatively display, on the user interaction interface, the medical data which is related to the physiological parameter(s) and an operation interface for adjusting the control parameter, which parameters are in a same group, the user interaction apparatus is further configured to:
display the medical data; and
in response to a selection instruction for the medical data related to the physiological parameter(s) that is(are) in a target group, display the operation interface for adjusting the control parameter, which control parameter is also in the target group.

32. The system according to claim 24, **characterized in that**, in order to associatively display, on the user interaction interface, the medical data which is related to the physiological parameter(s) and an operation interface for adjusting the control parameter, which parameters are in a same group, the user interaction apparatus is further configured to:
display the medical data; and
when the medical data related to the physiological parameter(s) that is(are) in a target group, is detected to exceed a preset range, displaying the operation interface for adjusting the control parameter, which control parameter is also in the target group.

33. A display method for a medical device, **characterized in that**, comprising:
acquiring medical data of a medical object, wherein the medical data comprises data which is related to one or more physiological parameters of the medical object;
acquiring data for a control parameter to provide treatment to the medical object, wherein the medical device is configured to provide the treatment to the medical object based on the control parameter, so as to affect the medical data of the medical object;
determining a grouping of the physiological parameter(s) and the control parameter, wherein each group comprises at least one physiological parameter and at least one control parameter which is capable of affecting medical data which is related to said physiological parameter;
providing a user interaction interface; associatively displaying, on the user interaction interface, the medical data which is related to the physiological parameter(s) and an operation interface for adjusting the control parameter, which parameters are in a same group, or associatively displaying, on the user interaction interface, the medical data which is related to the physiological parameter(s) and the data for the control parameter, which parameters are in a same group;
when an adjustment instruction for the control parameter is obtained, adjusting the control parameter according to the adjustment instruction.

34. A medical device, **characterized in that**, comprising:
a monitoring component, which is configured to acquire medical data of a medical object, wherein the medical data comprises data which is related to one or more physiological parameters of the medical object;
a treatment component, which is configured to provide treatment to the medical object based on a control parameter, so as to affect the medical data;
a user interaction apparatus, which is configured to provide a user interaction interface;
a control apparatus, which is configured to:
control the user interaction apparatus to associatively display, on the user interaction interface, according to a grouping of the physiological parameter(s) and the control parameter, the medical data which is related to the physiological parameter(s) and an operation interface for adjusting the control parameter, which parameters are in a same group, or to associatively display, on the user interaction interface, according to a grouping of the physiological parameter(s) and the control parameter, the medical data which is related to the physiological parameter(s) and data for the control parameter, which parameters are in a same group; wherein each group comprises at least one physiological parameter and at least one control parameter which is capable of affecting medical data which is related to said physiological parameter; and
when an adjustment instruction for the control parameter is obtained, adjust the control parameter according to the adjustment instruction.
